# EUROPÄISCHE PATENTSCHRIFT

(11) **EP 3 777 742 B1**
(45) Veröffentlichungstag und Bekanntmachung des Hinweises auf die Patenterteilung: **24.12.2025**
(21) Anmeldenummer: 20182214.5
(22) Anmeldetag: 25.06.2020
(51) Int. Cl.: A61B 18/14, A61B 18/16

(54) **VOR KURZSCHLUSS GESCHÜTZTES ELEKTRODENINSTRUMENT UND RESEKTOSKOP**
ELECTRODE INSTRUMENT PROTECTED AGAINST SHORT CIRCUIT AND RESECTOSCOPE
INSTRUMENT À ÉLECTRODES À PROTECTION CONTRE LES COURTS-CIRCUITS ET RÉSECTOSCOPE

(30) Priorität: 12.08.2019 DE 102019121674
(43) Veröffentlichungstag der Anmeldung: 17.02.2021
(73) Patentinhaber: Olympus Winter & Ibe GmbH, 22045 Hamburg (DE)
(72) Erfinder: BROCKMANN, Christian, 21279 Hollenstedt (DE); KNOPF, Christoph, 23617 Stockelsdorf (DE)
(74) Vertreter: Hoener, Matthias

(56) Entgegenhaltungen:
- EP-A1- 2 767 250
- DE-C1- 10 028 850
- US-A- 5 827 274
- US-A1- 2003 130 655
- US-A1- 2014 378 965

## Beschreibung

Die Erfindung betrifft Elektrodeninstrumente zur Verwendung in einem Resektoskop der im Oberbegriff des Anspruchs 1 genannten Art sowie ein Resektoskop der im Oberbegriff des Anspruchs 6 genannten Art.

Elektrodeninstrumente und Resektoskope der gattungsgemäßen Art werden vor allem in der Urologie bei elektrochirurgischen Arbeiten in der Blase und der Urethra verwendet. Sie werden üblicherweise zur Resektion und Vaporisation von Gewebe, zum Beispiel von Gewebe im unteren Harntrakt, verwendet. Dazu umfassen die Resektoskope ein gattungsgemäßes Elektrodeninstrument, das innerhalb des Resektoskopschaftes längsverschiebbar gelagert ist und nach dem Einführen des Resektoskops mit seinem distalen Arbeitsende aus dem distalen Ende des Schaftes hervorgeschoben werden kann. Das Elektrodeninstrument umfasst an seinem distalen Arbeitsende eine elektrochirurgische Elektrode, zum Beispiel in Form eines Loops (Schlinge) oder Vaporisationsknopfes (z. B. PlasmaButtons). Solche Instrumente sind zum Beispiel die OES PRO Resektoskope (Olympus) oder andere Dauerspül-Resektoskope nach Iglesias.

Mittels dieser Elektrodeninstrumente können die Prostata oder Teile der Prostata resiziert werden, beispielsweise bei Vorliegen einer benignen Prostatahyperplasie (BPH), d.h. einem überschießendem Wachstum von Prostatazellen. Eine solche Prostatavergrößerung wird im Laufe eines normalen Alterungsprozesses durch Abfall des Androgen- und Ansteigen des Östrogenspiegels im Blut begünstigt. In jüngerer Zeit wurde als Alternative zur Gewebsresektion das Prostatagewebe durch Implantate komprimiert. Die Kompression wurde durch Implantate erreicht, die an beiden Enden ankerartig ausgebildet sind und in der Lage sind, das Prostatagewebe komprimiert zu halten (UroLift^{®} - System). Durch die Kompression des Gewebes wird zumindest temporär der Druck auf die Urethra reduziert, sodass Urin abfließen kann. Ein entsprechendes System ist beispielsweise in der EP 2 164 427 und US 7,645,286 B2 beschrieben.

Es hat sich jedoch gezeigt, dass auch nach solchen komprimierenden Eingriffen zu einem späteren Zeitpunkt durch fortwährendes Gewebswachstum ein Resektionseingriff am Prostatagewebe erforderlich werden kann. Werden diese Eingriffe mit üblichen bipolaren Elektrodeninstrumenten durchgeführt, besteht das Risiko das Resektoskop zu beschädigen, wenn die leitenden Implantate den isolierenden Abstand zwischen der Arbeitselektrode und Schaft, Gabelrohr oder Optik überbrücken. Bei besonders dünnen Elektroden, wie Schlingenelektroden, besteht das zusätzliche Risiko, dass der Draht durch eine lokal erhöhte Stromdichte bei Kontakt mit dem Implantat aufschmilzt und reißt.

Aus der EP 2 767 250 A1 ist ein Resektoskop bekannt, welches eine Schneidelektrode aufweist, die mit Rückführelektroden verbunden ist. Am Übergang zur Schneidelektrode sind die Rückführelektroden mit einer Isolierschicht versehen. Außerdem kann oberhalb der abwärts gerichteten Schneidelektrode ein nichtleitendes Schild vorgesehen sein.

In der DE 100 28 850 C1 ist ein HF-resektoskopisches Instrument zum Schneiden von Körpergewebe gezeigt, mit schlingenförmiger Schneidelektrode und flächiger Neutralelektrode an einem Schlingenträger und einem Isolatorkörper an der Neutralelektrode.

Die US 5 827 274 A offenbart eine Vaporisationselektrode mit teilsphärischen Oberflächen. Zur Elektrode hinführende Leiter können außenseitig isoliert sein.

Die US 2014/0 378 965 A1 offenbart ein Resektoskop mit einem Elektrodeninstrument. Dieses ist vorgesehen mit einer monopolaren aktiven Elektrode, welche in Figuren dargestellt ist, oder in nur im Text erwähnter bipolarer Konfiguration mit einer Gegenelektrode. Auf der Arbeitselektrode ist eine isolierende Keramikschicht vorgesehen. Außerdem kann ein Teilbereich der Arbeitselektrode mit einer isolierenden Bedeckung versehen sein. Schließlich können zwei alternativ ansteuerbare Arbeitselektroden, nämlich eine Vaporisationselektrode und eine Koagulationselektrode durch eine Isolierschicht getrennt sein.

Die US 2003/0 130 655 A1 offenbart ein elektrochirurgisches Instrument für die Koagulation von Gewebe. Eine hochfrequente Spannung liegt zwischen einer äußeren aktiven Elektrode und einer Gegenelektrode an. Linear zwischen den Elektroden ist ein überbreiter elektrisch isolierender Elektrodenhalter angeordnet.

Es besteht daher Bedarf an Elektrodeninstrumenten, für die das Risiko einer Beschädigung des Instruments bei Eingriffen an Prostatagewebe, das Implantate erhält, reduziert ist.

### Beschreibung

Gelöst wird diese Aufgabe durch ein bipolares Elektrodeninstrument mit den Merkmalen von Anspruch 1 und ein Resektoskop mit den Merkmalen von Anspruch 6. Erfindungsgemäß ist insbesondere vorgesehen, die Arbeitselektrode und Gegenelektrode derart voneinander zu beabstanden bzw. derart anzuordnen, dass der Abstand zwischen ihnen nicht mittels eines Leiters überbrückbar ist, der eine Länge von 6 mm oder weniger aufweist. Diese Länge entspricht der Länge von End- bzw. Ankerstücken derzeit verwendeter, oben erwähnter Prostataimplantate.

In einem ersten Aspekt betrifft die Erfindung ein bipolares Elektrodeninstrument zur Verwendung in einem Resektoskop, wobei das Elektrodeninstrument einen langgestreckten Schaftabschnitt aufweist, durch den hindurch sich ein erster Leiter erstreckt, der am distalen Ende des Elektrodeninstruments eine mit Hochfrequenzstrom beaufschlagbare Arbeitselektrode bildet, wobei sich ferner durch den Schaftabschnitt ein zweiter Leiter erstreckt, der im distalen Endbereich des Elektrodeninstruments eine Gegenelektrode bildet, dadurch gekennzeichnet, dass die Arbeitselektrode und die Gegenelektrode räumlich derart voneinander getrennt sind, dass der Abstand zwischen ihnen nicht mittels eines geraden Leiters überbrückbar ist, der eine Länge von 6 mm oder weniger aufweist.

In einem zweiten, verwandten Aspekt betrifft die Erfindung ein Resektoskop für die endoskopische Chirurgie mit einem Hüllrohr, dadurch gekennzeichnet, dass innerhalb des Hüllrohres ein erfindungsgemäßes Elektrodeninstrument längsverschiebbar gelagert ist.

Das erfindungsgemäße Resektoskop ist für verschiedene Eingriffe in der endoskopischen Chirurgie geeignet, insbesondere in der elektrochirurgischen Chirurgie. So kann das Resektoskop beispielsweise für die Prostata-Resektion verwendet werden. Gleichzeitig kann das Resektoskop aber auch für eine Vielzahl anderer Operationen eingesetzt werden, zum Beispiel für Blasen-Resektionen.

In üblicher Ausbildungsweise weist das erfindungsgemäße Resektoskop einen rohrartigen Schaft auf. Der Resektoskopschaft umfasst ein langgestrecktes Hüllrohr. Neben dem Schaft umfasst das Resektoskop zum Halten und Bedienen ein Griffsystem, das üblicherweise aus zwei Griffteilen besteht.

Das erfindungsgemäße bipolare Elektrodeninstrument wird als Durchgangsinstrument in einem solchen Resektoskop verwendet. Dazu ist das Elektrodeninstrument längsverschiebbar in dem Resektoskop gelagert, vorzugsweise innerhalb des Hüllrohres des Resektoskops. Das Elektrodeninstrument kann außerhalb eines in dem Hüllrohr verlaufenden Innenrohrs, d.h. zwischen Hüllrohr und Innenrohr, angeordnet sein oder innerhalb des Innenrohres. Zusätzlich zu dem Elektrodeninstrument können weitere Durchgangsinstrumente in dem Schaft des Resektoskops angeordnet sein. Üblicherweise umfasst das Resektoskop zusätzlich beispielsweise eine stabförmige Optik, ein Beleuchtungsmittel und/oder ein Spülsystem.

Das Elektrodeninstrument weist in üblicher Ausbildungsweise einen langgestreckten Schaftabschnitt auf, der im Inneren des Resektoskopschaftes, wie oben beschrieben, längsverschiebbar und optional um seine Längsachse rotierbar angeordnet ist. Das proximale Ende des Schaftabschnitts ist funktional mit einem Schlitten des Transporteurs des Resektoskops verbunden, der mittels des Handgriffs betätigt werden kann. Am distalen Ende des Elektrodeninstruments ist eine mit Hochfrequenzstrom beaufschlagbare Arbeitselektrode ausgebildet.

Das Elektrodeninstrument kann, insbesondere im Schaftabschnitt, einen oder zwei Tragarme aufweisen, an dem oder zwischen denen die Arbeitselektrode und die Gegenelektrode gehalten werden können. So kann die Arbeitselektrode beispielsweise sicher zwischen den distalen Enden zweier Tragarme oder an dem distalen Ende des einen Tragarmes gehalten werden. Dabei ist die Arbeitselektrode vorzugsweise so angeordnet, dass die Sicht auf die Eingriffsstelle nicht in erheblichem Maße beeinträchtigt wird.

Durch den bzw. die Tragarme bzw. den Schaftabschnitt hindurch erstrecken sich zwei Leiter. Der erste Leiter bildet am distalen Ende des Elektrodeninstruments eine mit Hochfrequenzstrom beaufschlagbare Arbeitselektrode (auch: Aktivelektrode). Der zweite Leiter bildet im distalen Endbereich des Elektrodeninstruments eine Gegenelektrode (auch: Neutralelektrode). Die Arbeitselektrode ist somit mit dem ersten Leiter und die Gegenelektrode mit dem zweiten Leiter elektrisch leitend verbunden. Die Elektroden sind dagegen nicht miteinander oder mit dem jeweils anderen Leiter elektrisch leitend verbunden. Auf diese Weise fließt der Strom während eines chirurgischen Eingriffs zwischen den beiden Elektroden durch das elektrisch leitfähige Gewebe bzw. die Flüssigkeit das bzw. die sich zwischen den beiden Elektroden befindet. Das Elektrodeninstrument ist somit erfindungsgemäß ein bipolares Elektrodeninstrument. Die Vorteile von bipolaren gegenüber monopolaren Instrumenten sind Fachleuten hinreichend bekannt. Die beiden Leiter sind, in der Regel über Anschlusskabel, mit zwei Polen eines Hochfrequenzgenerators verbunden.

Beide Leiter sind elektrisch leitfähig und können beispielsweise als Leiterdraht ausgebildet sein. Im Schaftabschnitt, mit Ausnahme der Elektroden, kann der Leiterdraht z. B. gegenüber dem Resektoskopschaft elektrisch isoliert sein. Zu diesem Zweck können der oder die Tragarme in der Regel eine den betreffenden Leiter umgebene Isolierhülle, d.h. eine elektrisch isolierende Hülle, aufweisen. Die Isolierhülle kann eine Hohlzylinderform aufweisen, in deren Inneren der bzw. die Leiter verlaufen. Es ist auch denkbar, nur einen der Leiter mit einer Isolierhülle zu umgeben und den anderen nicht oder nur teilweise. Entsprechende Lösungen sind Fachleuten auf dem Gebiet bekannt. Am proximalen Ende der Tragarme ist der Leiter über den proximalen Abschnitt des Resektoskops, wie oben beschrieben, mit einer Stromquelle verbunden.

Die Gegenelektrode ist von der Arbeitselektrode beabstandet. Dieser Abstand ist hinreichend, um die beiden Elektroden voneinander elektrisch zu isolieren. Hierzu ist in der Regel ein Abstand von etwa 2 mm ausreichend. Die Gegenelektrode kann somit 2 mm oder mehr von der Arbeitselektrode entfernt sein, vorzugsweise 3 mm oder mehr, besonders bevorzugt 4 mm oder mehr. Vorzugsweise ist die Gegenelektrode longitudinal, d.h. entlang der Längsachse des Elektrodeninstruments, auf gleicher Höhe oder proximal von der Arbeitselektrode angeordnet.

Die Arbeitselektrode kann bei Hochfrequenzbeaufschlagung durch Gewebe schneiden und kann zum Beispiel dazu benutzt werden, einen Tumor oder überschüssiges Prostatagewebe abzutragen. Dazu muss bei eingeschaltetem Hochfrequenzgenerator das Elektrodeninstrument, insbesondere die Arbeitselektrode, in geeigneter Weise schneidend bewegt werden.

Die Arbeitselektrode kann verschiedene Formen aufweisen, die auf dem Gebiet der Hochfrequenzchirurgie bekannt sind. Sie kann beispielsweise als Schlingenelektrode, Bandelektrode oder Vaporisationsknopf ausgebildet sein. Ein Vaporisationsknopf ist eine teilsphärische, meist halbsphärische, Arbeitselektrode, die auch als PlasmaButton bezeichnet wird. Vorzugsweise ist die Arbeitselektrode ein solcher teilsphärischer Vaporisationsknopf.

Erfindungsgemäß sind die Arbeitselektrode und die Gegenelektrode räumlich derart voneinander getrennt, dass der Abstand zwischen ihnen nicht mittels eines geraden, zum Beispiel stabförmigen, Leiters überbrückbar ist, der eine Länge von 6 mm oder weniger aufweist. Es wird mit anderen Worten durch die Anordnung der Arbeits- und Gegenelektrode verhindert, dass zwischen den beiden durch Berührung der beiden Elektroden gleichzeitig mit einem weiteren Leiter eine leitfähige Verbindung zwischen den beiden Elektroden und damit ein Kurzschluss entstehen kann. Unter einer Überbrückung des Abstands zwischen den beiden Elektroden wird hierin demnach die Herstellung einer elektrisch leitfähigen Verbindung zwischen den beiden Elektroden verstanden. Dieser weitere Leiter, der nicht Teil des Elektrodeninstruments oder des Resektoskops ist, kann beispielsweise ein Teil eines elektrisch leitfähigen Implantats sein. Der Leiter kann daher auch als instrumentenexterner Leiter bezeichnet werden. Der instrumentenexterne Leiter ist nicht Gewebe oder Flüssigkeit, sondern in der Regel aus einem elektrisch leitfähigen Metall oder einer Legierung, die ein solche enthält. Der instrumentenexterne Leiter ist im Wesentlichen gerade und weist keine größeren Krümmungen auf.

6 mm entsprechen der kürzesten Ankerlänge derzeit verwendeter Prostataimplantate. In vielen Fällen werden jedoch Implantate mit einer Ankerlänge von 8 mm verwendet. In einer Ausführungsform der Erfindung sind die Arbeitselektrode und die Gegenelektrode daher räumlich derart voneinander getrennt, dass der Abstand zwischen ihnen nicht mittels eines geraden, zum Beispiel stabförmigen, Leiters überbrückbar ist, der eine Länge von 8 mm oder weniger aufweist.

Die Gegenelektrode ist im distalen Endbereich des Elektrodeninstruments angeordnet. Dies bedeutet, dass die Gegenelektrode sich beispielsweise im distalen Drittel, vorzugsweise im distalen Fünftel, stärker bevorzugt im distalen Zehntel des Elektrodeninstruments befindet, nicht aber unmittelbar am distalen Ende des Instruments angeordnet sein muss. In der Regel wird die Gegenelektrode relativ dicht bei der Arbeitselektrode angeordnet sein, aber von dieser elektrisch isoliert.

In bevorzugten Ausführungsformen wird erfindungsgemäß ein Kurzschluss durch einen instrumentenexternen Leiter dadurch verhindert, dass der Abstand zwischen Arbeitselektrode und Gegenelektrode mehr als 6 mm beträgt. Da die maximale Länge der Anker der kleinsten üblichen Implantate 6 mm beträgt, kann auf diese Weise das Risiko für einen Kurzschluss zwischen Arbeits- und Gegenelektrode hinreichend reduziert werden. Der Abstand zwischen Arbeitselektrode und Gegenelektrode kann mehr als 6 mm, mehr als 8 mm, mehr als 9 mm, mehr als 10 mm, mehr als 11 mm, mehr als 12 mm, mehr als 13 mm, mehr als 14 mm, oder mehr als 15 mm betragen. Vorzugsweise ist der Abstand mehr als 8 mm, da dies der Länge der Anker häufig verwendeter Implantate entspricht. Mit anderen Worten beträgt der Abstand zwischen der Arbeitselektrode und der Gegenelektrode zwischen 6 mm und 20 mm, zwischen 8 mm und 20 mm, zwischen 10 mm und 20 mm, zwischen 11 mm und 20 mm, zwischen 12 mm und 20 mm, zwischen 13 mm und 20 mm, zwischen 14 mm und 20 mm, oder zwischen 15 mm und 20 mm. Auch ein Maximalabstand von 15 mm kann vorgesehen sein. Es versteht sich, dass der Begriff "zwischen" bei diesen Bereichsangaben, die genannte Unter- und Obergrenze ausschließt. Die genannten Abstände sind länger als derzeit übliche Abstände zwischen der Arbeitselektrode und der Gegenelektrode, die in der Regel bei etwa 4 mm liegen. Erfindungsgemäß ist somit beispielsweise der Tragarm bzw. die Tragarme entsprechend verlängert. Es ist aber auch denkbar, die Gegenelektrode an einem Tragarm mit üblicher Länge weiter proximal anzuordnen.

Alternativ oder zusätzlich zu der beschriebenen Abstandsverlängerung kann erfindungsgemäß ein Kurzschluss zwischen der Arbeitselektrode und der Gegenelektrode verhindert werden, indem ein Isolator derart zwischen den beiden Elektroden platziert ist, dass mittels eines instrumentenexternen, geraden Leiters keine elektrisch leitfähige Verbindung zwischen den beiden Elektroden herstellbar ist. In diesen Ausführungsformen ist, mit anderen Worten, räumlich zwischen Arbeitselektrode und Gegenelektrode ein Isolator angeordnet, der alle zwischen Arbeitselektrode und Gegenelektrode möglichen Verbindungsgeraden, die eine Länge von 6 mm oder weniger aufweisen, schneidet. In bevorzugten Ausführungsformen schneidet der Isolator alle zwischen Arbeitselektrode und Gegenelektrode möglichen Verbindungsgeraden, die eine Länge von 8 mm oder weniger aufweisen. Stärker vorzugsweise schneidet der Isolator nicht nur alle Verbindungsgeraden, die eine Länge von 6 mm oder weniger bzw. 8 mm oder weniger aufweisen, sondern alle zwischen Arbeitselektrode und Gegenelektrode möglichen Verbindungsgeraden.

Als Verbindungsgeraden werden hierin alle Geraden angesehen, die von einem äußeren Punkt der Arbeitselektrode zu einem äußeren Punkt der Gegenelektrode verlaufen. Als 'äußere Punkte' gelten alle Punkte auf der Außenwand der Elektroden. Es werden nur solche Geraden als Verbindungsgeraden angesehen, die bzw. deren Strecke effektiv zur elektrisch leitfähigen Verbindung von Arbeitselektrode und Gegenelektrode mittels eines geraden Leiters genutzt werden könnten. Bevorzugt werden somit insbesondere solche Verbindungsgeraden durch den Isolator geschnitten, auf denen prinzipiell zur Herstellung einer elektrischen Verbindung zwischen Arbeitselektrode und Gegenelektrode ein gerader Leiter platziert werden könnte. Es versteht sich, dass die Verbindungsgeraden im Sinne der Erfindung virtuell sind, d.h. keine realen technischen Verbindungselemente. Geraden, die von einem oder mehreren anderen isolierenden Elementen des Elektrodeninstruments geschnitten werden und auf denen daher keine elektrisch leitfähige Verbindung hergestellt werden könnte, werden nicht als Verbindungsgeraden angesehen. Solche anderen isolierenden Elemente können beispielsweise der oder die Tragarme sein, sofern diese von einer Isolierhülle umgeben sind.

Der Isolator schneidet, wie oben beschrieben, die Verbindungsgeraden. 'Schneiden' bedeutet im üblichen Wortsinne, dass der Isolator die Verbindungsgeraden in ihrem Verlauf kreuzt bzw. in ihrem Verlauf schneidet.

Der Isolator kann ein oder mehrere Isolierelemente umfassen, beispielsweise 1, 2, 3, 4, 5, 6 oder mehr. Die Isolierelemente können im Isolator einander unmittelbar benachbart sein, oder mit einigem Abstand voneinander angeordnet sein.

Fachleuten sind geeignete Materialien, aus denen der Isolator aufgebaut sein kann, bekannt. Da der Isolator elektrisch isolierend ist, können im Wesentlichen alle elektrisch isolierenden Materialien verwendet werden, wobei es aufgrund der Nähe zur Arbeitselektrode bevorzugt ist, plasmabeständige Materialien zu verwenden. Der Isolator soll durch die mit Hochfrequenzstrom beaufschlagte und erhitzte Arbeitselektrode nicht beschädigt werden. Zu diesem Zweck ist der Isolator vorzugsweise vollständig, mindestens aber in einem Maße, das seine Isolierfähigkeit gewährleistet, aus einem elektrisch nicht leitfähigen, d.h. elektrisch isolierenden, Material ausgebildet. Solche Materialien sind Fachleuten bekannt und umfassen beispielsweise Keramiken und Kunststoffe. Geeignete Kunststoffe können beispielsweise ausgewählt sein aus der Gruppe bestehend aus Fluorpolymeren und Cycloolefin-Copolymeren.

Der Isolator kann von den beiden oder einer der Elektroden beabstandet sein. In bestimmten Ausführungsformen ist es allerdings bevorzugt, dass der Isolator direkt an eine oder beide Elektroden angrenzt, zum Beispiel angrenzend an beide Elektroden zwischen den beiden Elektroden angeordnet ist. So kann der Isolator beispielsweise direkt an die Arbeitselektrode und/oder direkt an die Gegenelektrode angrenzen. Es ist z. B. denkbar, den Isolator an der nicht-sphärischen Seite eines Vaporisationsknopfes anzuordnen, d.h. der Seite der Arbeitselektrode, die während eines Eingriffs von dem Gewebe abgewandt ist. Die Gegenelektrode kann dann proximal von der Arbeitselektrode angeordnet sein oder auf der Seite des Isolators, die von der Arbeitselektrode abgewandt ist.

Der Isolator kann zu diesem Zweck beispielsweise plattenförmig ausgebildet sein, d.h. eine flache und ebene Form aufweisen, die vorzugsweise in einer Ebene überall gleich dick ist. Auf diese Weise kann der Isolator material- und platzsparend die Verbindungsgeraden kreuzen und gleichzeitig in Richtung der Verbindungsgeraden möglichst wenig Platz einnehmen.

Der Isolator kann beispielsweise einen flachen quader- oder L-förmigen Querschnitt aufweisen. Ein Isolator mit einem L-förmigem Querschnitt ist insbesondere zur Abschirmung der Arbeitselektrode geeignet, wenn es sich bei dieser um einen Vaporisationsknopf handelt. Der Isolator mit einem L-förmigen Querschnitt kann dann derart platziert werden, dass der Vaporisationsknopf zweiseitig von dem Isolator umschlossen ist, wobei eine Seite vorzugsweise zur Befestigung des Isolators die dem Tragarm zugewandte Seite der Arbeitselektrode ist und die andere Seite die an anderer Stelle hierin beschriebenen Verbindungsgeraden schneidet.

Ist der Isolator alternativ mit einem flachen quaderförmigen Querschnitt ausgebildet, dann kann er beispielsweise angrenzend an die Arbeitselektrode auf der Seite der Arbeitselektrode aufliegen, die dem Tragarm zugewandt ist. In dieser Ausführungsform könnte die Gegenelektrode wiederum unmittelbar auf der Seite des Isolators angeordnet sein, die dem Tragarm zu- und der Arbeitselektrode abgewandt ist. Die Gegenelektrode und Arbeitselektrode werden dann radial nicht über den Isolator hinausragen, insbesondere nicht derart über den Isolator hinausragen, dass Verbindungsgeraden zwischen Arbeits- und Gegenelektrode entstehen, über die mittels eines geraden Leiters eine elektrisch leitfähige Verbindung hergestellt werden könnte. In dieser Ausführungsform können die Arbeits- und Gegenelektrode sehr dicht beieinander platziert werden, sodass die Stromstrecke bei Verwendung der Elektrode kurz ist.

Es ist auch denkbar, dass die Arbeitselektrode und der Isolator auf einer Seite des Elektrodeninstruments angeordnet sind und die Gegenelektrode auf der, mit Referenz zur Längsachse des Elektrodeninstruments, gegenüberliegenden Seite des Elektrodeninstruments angeordnet ist. Durch diese räumliche Trennung von Arbeits- und Gegenelektrode gibt es keine Verbindungsgeraden im Sinne der Erfindung zwischen den Elektroden. Da die Arbeitselektroden oftmals an einem im distalen Endbereich abgewinkelten Tragarm oder Tragarmen angeordnet sind, kann die Gegenelektrode beispielsweise an der Seite des Tragarms angeordnet sein, die der Abwinkelungsrichtung, mit Referenz zur Längsachse des Elektrodeninstruments, gegenüberliegt, und in einem Abschnitt des Tragarms, in dem der Tragarm vorzugsweise im Wesentlichen parallel zur Längsachse des Elektrodeninstruments verläuft.

### Kurze Beschreibung der Figuren

In den Zeichnungen sind Ausführungsbeispiele der Offenbarung schematisch dargestellt. Es zeigen:
- Fig. 1: eine schematische Seitenansicht eines erfindungsgemäßen Resektoskops;
- Fig. 2: eine schematische Seitenansicht eines erfindungsgemäßen Elektrodeninstruments;
- Fig. 3: eine schematische Seitenansicht des distalen Endbereichs eines Elektrodeninstruments aus dem Stand der Technik, wobei das Elektrodeninstrument eine Vaporisationsknopf- (Button-) Arbeitselektrode aufweist;
- Fig. 4: eine schematische Seitenansicht des distalen Endbereichs eines nicht erfindungsgemäßen Elektrodeninstruments, wobei das Elektrodeninstrument eine Vaporisationsknopf- (Button-) Arbeitselektrode aufweist und der distale Endbereich des Tragarms gegenüber dem Stand der Technik verlängert ist;
- Fig. 5: eine schematische Seitenansicht des distalen Endbereichs eines erfindungsgemäßen Elektrodeninstruments, wobei das Elektrodeninstrument eine Vaporisationsknopf- (Button-) Arbeitselektrode und einen Isolator mit einem L-förmigen Querschnitt aufweist, der die Verbindungsgeraden zwischen Arbeitselektrode und Gegenelektrode schneidet;
- Fig. 6: eine schematische Seitenansicht des distalen Endbereichs eines nicht erfindungsgemäßen Elektrodeninstruments, wobei das Elektrodeninstrument eine Vaporisationsknopf- (Button-) Arbeitselektrode und einen Isolator auf einer Seite des Elektrodeninstruments aufweist und auf der, mit Referenz zur Längsachse des Elektrodeninstruments, gegenüberliegenden Seite des Elektrodeninstruments eine Gegenelektrode aufweist; und
- Fig. 7: eine schematische Seitenansicht des distalen Endbereichs eines nicht erfindungsgemäßen Elektrodeninstruments, wobei das Elektrodeninstrument eine Vaporisationsknopf- (Button-) Arbeitselektrode und einen Isolator mit einem rechteckigen Querschnitt aufweist, der angrenzend zwischen Arbeits- und Gegenelektrode angeordnet ist.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

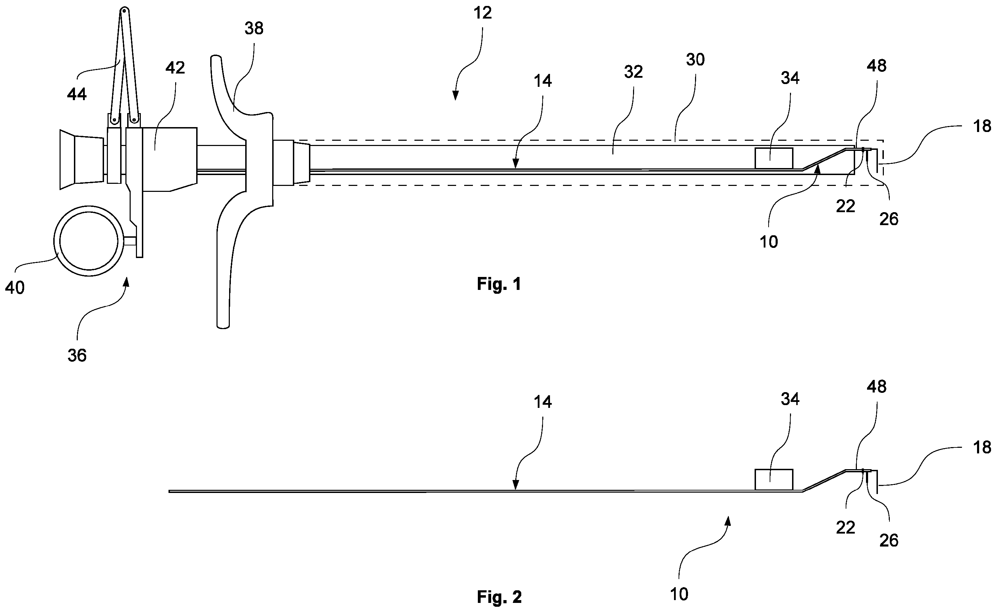

Fig. 1 zeigt eine schematische Seitenansicht eines erfindungsgemäßen Resektoskops 12, Fig. 2 das darin angeordnete Elektrodeninstrument 10. Das in beiden Figuren dargestellte Elektrodeninstrument 10 weist eine Arbeitselektrode 18 und eine Gegenelektrode 22 auf, sowie einen Isolator 26, der durch externe Leiter 24 verursachbare Kurzschlüsse verhindert.

Das Resektoskop 12 weist einen Schaft auf, der ein Hüllrohr 30 umfasst, das in Fig. 1 gestrichelt dargestellt ist. Innerhalb des Hüllrohres 30 verläuft ein Innenrohr 32 und innerhalb des Innenrohres 32 das Elektrodeninstrument 10. Darüber hinaus können weitere, hier nicht dargestellte, Elemente im Inneren des Hüllrohres 30 verlaufen, wie zum Beispiel eine Optik, Lichtleitfaserbündel und/oder Spülrohre.

Das Elektrodeninstrument 10 ist längsverschiebbar in dem Innenrohr 32 angeordnet und gegen Querverschiebungen, d.h. Verschiebungen in radiale Richtung, durch zwei Führungselemente 34 geschützt. Die Führungselemente 34, von denen in den Fig. 1 und 2 jeweils nur eines sichtbar ist, sind formkomplementär zur Innenwandung des Innenrohres 32 als Führungsbleche ausgebildet, die gemeinsam eine teilzylindrische Form einnehmen.

Das Elektrodeninstrument 10 kann durch Betätigung eines Griffteils 40 zwangsgeführt axial in distale und proximale Richtung bewegt werden. Dabei kann es über das distale Ende des Innenrohres 32 und das distale Ende des Hüllrohres 30 hinausgeschoben werden. So wird es dem Operateur ermöglicht, auch weiter von der Resektoskopspitze entferntes Gewebe zu manipulieren. Zu diesem Zweck sind ferner Innenrohr 32 und/oder Elektrodeninstrument 10 um ihre Längsachse drehbar gelagert.

Das Elektrodeninstrument 10 weist an seinem distalen Ende eine Arbeitselektrode 18 auf, die in der gezeigten Ausführungsform als Schneidschlinge ausgebildet ist. Mittels der Arbeitselektrode 18 kann Gewebe durch elektrochirurgische Ablation entfernt werden. Hierbei wird an die Arbeitselektrode 18 eine hochfrequente, elektrische Spannung angelegt, um Gewebe zu zerschneiden.

Das Elektrodeninstrument 10 weist in seinem distalen Endbereich ferner eine Gegenelektrode 22 auf und ist somit ein bipolares Elektrodeninstrument, bei dem zwei Elektroden getrennt voneinander an zwei Pole eines hier nicht dargestellten Hochfrequenzgenerators angeschlossen sind. Zwischen diesen Elektroden kann Strom fließen, wenn sie beispielsweise gemeinsam in einer Flüssigkeit sind, zum Beispiel der Flüssigkeit der Harnblase. Die Gegenelektrode 22 ist im Abstand, wie gezeigt zum Beispiel in proximalen Abstand, zur Arbeitselektrode 18 angeordnet. Beide Elektroden sind an dem bzw. den Tragarmen 48 des Elektrodeninstruments 10 angeordnet.

Der oder die Tragarme 48 des Elektrodeninstruments 10 sind stabförmig ausgebildet. Im Inneren des oder der Tragarme 48 verlaufen ein erster elektrischer Leiter 16 und ein zweiter elektrischer Leiter 24 (nicht dargestellt), die in ihren distalen Endbereichen die Arbeitselektrode bzw. die Gegenelektrode bilden. Die Gegenelektrode 22 ist beispielsweise mit dem zweiten Leiter 20 elektrisch leitend verbunden, nicht aber mit dem ersten Leiter 16, zu dem nur eine isolierende Verbindung besteht. Die Gegenelektrode 22 ist somit nur mit dem zweiten Leiter 20 und die Arbeitselektrode 18 nur mit dem ersten Leiter 16 elektrisch verbunden.

Im distalen Endbereich des Elektrodeninstruments 10 befinden sich somit zwei elektrisch voneinander getrennte Elektroden 18 und 22, die getrennt über die Leiter 16 und 20 angeschlossen sind. Die Leiter 16 und 20 sind mit Anschlusskabeln mit getrennten Polen eines nicht dargestellten Hochfrequenzgenerators verbunden. Wird der Hochfrequenzgenerator eingeschaltet, so liegen unterschiedliche Spannungspole an der Arbeitselektrode 18 und der Gegenelektrode 22 und es fließt Strom zwischen ihnen durch die elektrisch leitfähige Körperflüssigkeit.

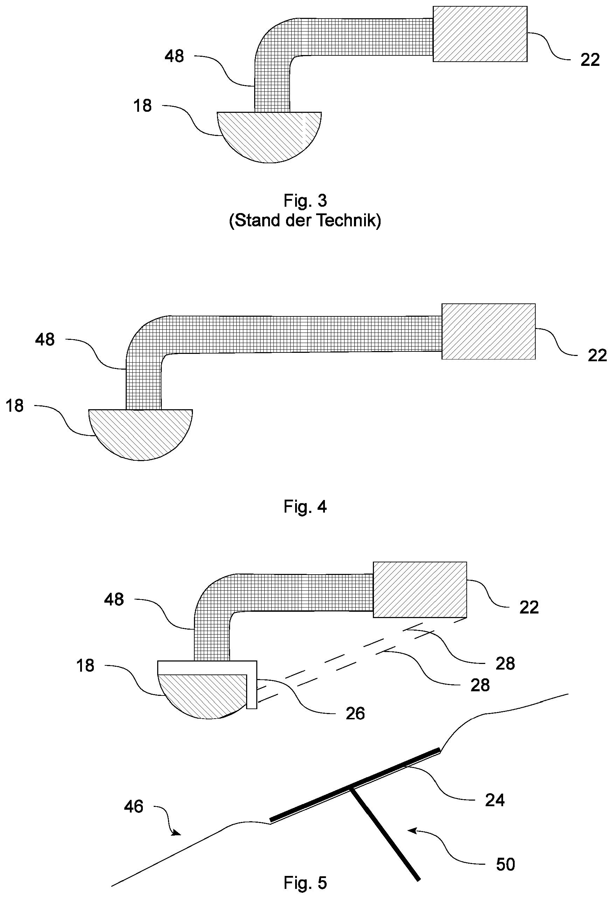

Bei Verwendung solcher bipolarer Instrumente besteht nach Anbringung von leitfähigen Implantaten, zum Beispiel an der Prostata, die Gefahr, dass ein von beiden Elektroden berührtes Stück eines Implantats zu einer leitenden Überbrückung und einem Kurzschluss zwischen Arbeitselektrode 18 und Gegenelektrode 22 führt. Daher ist erfindungsgemäß, wie in den Fig. 4 bis 7 gezeigt, vorgesehen, die Arbeitselektrode 18 und die Gegenelektrode 22 räumlich derart voneinander zu trennen, dass der Abstand zwischen ihnen nicht zwischen ihnen nicht mittels eines geraden, instrumentenexternen Leiters 24 überbrückbar ist, der eine Länge von 6 mm oder weniger aufweist.

Das dargestellte Resektoskop 12 weist einen passiven Transporteur auf, bei dem der Schlitten 42 durch Relativbewegung der proximal von dem Resektoskopschaft am Handgriff 36 angeordneten Griffteile 38, 40 zueinander gegen eine von einer Federbrücke 44 aufgebrachten Federkraft in distale Richtung gegen das distale, erste Griffteil 38 verschoben wird. Bei der Verschiebung des Schlittens 42 in distale Richtung gegen das Griffteil 38 wird das Elektrodeninstrument 10 in nicht dargestellter Weise zwangsgeführt nach distal verschoben. Bei einer Entlastung der Griffteile 38, 40 zwingt die von der Federbrücke 44 erzeugte Federkraft den Schlitten 42 zurück in seine Ruheposition, wobei der Schaft des Resektoskops 12 und somit auch das Elektrodeninstrument 10 in proximale Richtung gezogen wird. Bei der Rückverschiebung des Schlittens 42 kann ohne Handkraft des Operateurs, also passiv, ein elektrochirurgischer Eingriff mit dem Elektrodeninstrument 10 vorgenommen werden.

Fig. 3 zeigt eine schematische Seitenansicht des distalen Endbereichs eines Elektrodeninstruments 10 aus dem Stand der Technik, wobei das Elektrodeninstrument 10 eine Arbeitselektrode 18 aufweist, die als Vaporisationsknopf (Plasmabutton) ausgebildet ist. Die Arbeitselektrode 18 ist am distalen Ende eines Tragarms 48 angeordnet, wobei der Tragarm 48 in einem distalen Abschnitt, in dem die Arbeitselektrode 18 angeordnet ist, quer zur Längsachse des Elektrodeninstruments 10 verläuft und nach proximal abgewinkelt in einen Abschnitt des distalen Endbereichs übergeht, der parallel zur Längsachse des Elektrodeninstrument 10 angeordnet ist. In diesem Abschnitt, der parallel zur Längsachse ausgerichtet ist, ist die Gegenelektrode 22 angeordnet, die an der Außenhülle des Tragarms 48 angebracht ist. Der minimale Abstand zwischen der Arbeitselektrode 18 und der Gegenelektrode 22 beträgt 6 mm oder weniger, sodass die Gefahr besteht, dass durch leitende Implantatteile ein Kurzschluss zwischen Gegenelektrode 22 und Arbeitselektrode 18 verursacht wird. Der Tragarm 48 ist im Übrigen, d.h. außerhalb von Arbeitselektrode 18 und Gegenelektrode 22, elektrisch isoliert. In einer alternativen Ausführungsweise kann der Vaporisationsknopf von zwei Tragamen 48 gehalten werden. Auf diese Weise kann in einem Tragarm 48 der erste Leiter 16 und in dem anderen Tragarm 48 der zweite Leiter 24 geführt werden.

Fig. 4 zeigt eine schematische Seitenansicht des distalen Endbereichs eines nicht erfindungsgemäßen Elektrodeninstruments 10, wobei das Elektrodeninstrument 10 eine Arbeitselektrode 18 aufweist, die als Vaporisationsknopf ausgebildet ist. Der distale Endbereich des Tragarms 48 ist gegenüber dem in Fig. 3 gezeigten Stand der Technik verlängert, sodass der Abstand zwischen der Arbeitselektrode 18 und der Gegenelektrode 22 mehr als 6 mm beträgt. Dadurch besteht vorteilhafterweise nicht mehr die Gefahr eines potentiellen Kurzschlusses durch Berührung von Implantatteilen, da die Implantatteile mit denen das Resektoskop in Kontakt kommen kann, in der Regel nicht länger als 6 mm sind. Alternativ zu einer Verlängerung des Tragarmes 48 ist es auch denkbar, den Abstand zwischen der Arbeitselektrode 18 und der Gegenelektrode 22 auf andere Weise zu verlängern, beispielsweise durch eine proximalere Anordnung der Gegenelektrode 22 an dem Tragarm.

Fig. 5 zeigt eine schematische Seitenansicht des distalen Endbereichs eines erfindungsgemäßen Elektrodeninstruments 10, wobei das Elektrodeninstrument 10 eine Arbeitselektrode 18 aufweist, die als Vaporisationsknopf ausgebildet ist. Das Elektrodeninstrument 10 weist ferner einen Isolator 26 auf, der einen L-förmigen Querschnitt hat. Der Isolator 26 schneidet sämtliche virtuellen Verbindungsgeraden 28 zwischen Arbeitselektrode 18 und Gegenelektrode 22 auf denen es ansonsten möglich wäre, eine elektrisch leitende Verbindung mittels eines geraden externen Leiters 24 herzustellen. Ein solcher Leiter 24, der ein Ankerteil eines Implantats 50 ist, ist beispielhaft ebenfalls dargestellt. Das Implantat 50 ist im Gewebe 46 verankert. Es ist nur eine Seite des Implantats 50 gezeigt, das an beiden Enden T-förmig ausgebildet ist. Das Gewebe 46 kann beispielsweise Prostatagewebe sein, das komprimiert ist, um den Harnleiter eröffnet zu halten.

Es ist erkennbar, dass mittels des ankerartigen, geraden Endes des Implantats 50, dem Leiter 24, keine elektrisch leitende Verbindung durch gleichzeitige Berührung der Arbeitselektrode 18 und der Gegenelektrode 22 erzeugbar ist, da der Isolator 26 eine solche verhindert. Der Isolator 26 ist einerseits mit einer Seite angrenzend an die gewebeabgewandte Rückseite der Buttonelektrode angeordnet und anderseits mit einem zweiten Abschnitt, der durch seinen L-förmigen Querschnitt gebildet wird, proximal-seitlich der Arbeitselektrode 18 angeordnet. Dieser zweite Abschnitt des L-förmigen Isolators 26 scheidet alle potentiell mittels eines geraden Leiters 24 überbrückbaren Verbindungsgeraden 28.

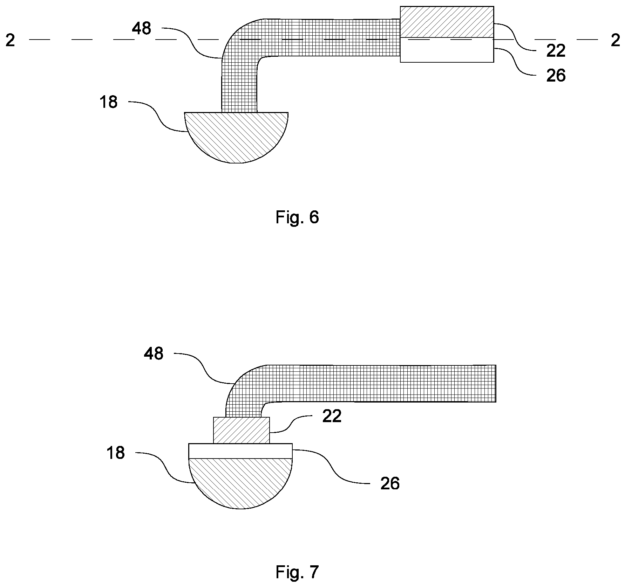

Fig. 6 zeigt eine schematische Seitenansicht des distalen Endbereichs eines nicht erfindungsgemäßen Elektrodeninstruments 10, wobei das Elektrodeninstrument 10 eine Arbeitselektrode 18 und einen Isolator 26 auf einer Seite des Elektrodeninstruments 10 aufweist und auf der, mit Referenz zur Längsachse 2-2 des Elektrodeninstruments 10, gegenüberliegenden Seite des Elektrodeninstruments 10 eine Gegenelektrode 22 aufweist. Die Position der Gegenelektrode 22 im distalen Endbereich des Elektrodeninstruments 10, d.h. der Abstand der Gegenelektrode 22 zu den distalen und proximalen Enden des Elektrodeninstruments 10, ist vergleichbar oder identisch zu dessen Position in Elektrodeninstrumenten 10 des Standes der Technik, wie dem in Fig. 3 gezeigten. Im Gegensatz zu diesen üblichen Instrumenten umfasst die Gegenelektrode 22 jedoch keine Teile, die auf der gleichen Seite des Elektrodeninstruments 10 angeordnet sind wie die Arbeitselektrode 18. Stattdessen ist an dieser Seite des Elektrodeninstruments 10 auf Höhe der Gegenelektrode 22 ein Isolator angeordnet. Dieser kann beispielsweise halbzylinderförmig um den Tragarm 48 herum ausgebildet sein und/oder einstückig mit der üblichen Isolierhülle des Tragarms 48. In bevorzugten Ausführungsformen ist der Isolator 26 durch eine elektrisch isolierende Beschichtung einer Seite der Tragarms 48 gebildet, zum Beispiel durch eine Beschichtung der Unterseite, die auf der gleichen Seite des Elektrodeninstruments 10 angeordnet ist, wie die Arbeitselektrode 18. Die Gegenelektrode 22 ist an der gegenüberliegenden Seite auf der Außenwandung des Tragarms 48 ausgebildet. Da der Tragarm 48 und der Isolator 26 zwischen Gegenelektrode 22 und Arbeitselektrode 18 angeordnet sind und der Isolator 26 alle zur potentiellen elektrischen Überbrückung verwendbaren Verbindungsgeraden 28 schneidet, besteht nicht die Gefahr eines Kurzschlusses zwischen Arbeitselektrode 18 und Gegenelektrode 22.

Fig. 7 zeigt eine schematische Seitenansicht des distalen Endbereichs eines nicht erfindungsgemäßen Elektrodeninstruments 10, wobei das Elektrodeninstrument 10 eine Arbeitselektrode 18 aufweist, die als Vaporisationsknopf ausgebildet ist. Der Tragarm 48 des Elektrodeninstruments 10, der die Arbeitselektrode 18 an seinem distalen Ende trägt, geht in seinem distalen Endbereich abgewinkelt in einen Abschnitt über, der in etwa quer zur Längsachse des Elektrodeninstruments 10 angeordnet ist (abgewinkelter Abschnitt). Neben der Arbeitselektrode 18 sind auch der Isolator 26 und die Gegenelektrode 22 in diesem abgewinkelten Abschnitt angeordnet. Arbeitselektrode 18 und Gegenelektrode 22 sind räumlich somit nicht weit voneinander entfernt. Die beiden Elektroden sind dennoch durch den zwischen den Elektroden angeordneten Isolator 26 sicher elektrisch voneinander isoliert. Alle drei Elemente sind im Wesentlichen equizentrisch um den mindestens einen Tragarm 48 herum angeordnet, d.h. die Mittelachse (Längsachse) des Tragarms 48 stellt bevorzugt auch die Mittelachse des Isolators 26 und der Gegenelektrode 22, vorzugsweise auch der Arbeitselektrode 18, dar. Ferner ist durch die Breite der drei Elemente sichergestellt, dass durch einen externen, geraden Leiter 24 keine elektrische Verbindung zwischen Arbeitselektrode 18 und Gegenelektrode 22 hergestellt werden kann. Dazu ist in der Regel mindestens eine der beiden Elektroden allseitig schmaler als der zwischen den Elektroden angeordnete Isolator 26. In dem gezeigten Beispiel ist dies die Gegenelektrode 22. Der Isolator 26 weist eine im Wesentlichen zylindrische Form auf. Es versteht sich, dass der Isolator 26 entsprechende Durchbrüche für den zur Arbeitselektrode 18 führenden Leiter 16 aufweist.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

**Bezugszeichenliste**

| | | | |
|---|---|---|---|
| 10 | Elektrodeninstrument | 48 | Tragarm |
| 12 | Resektoskop | 50 | Implantat |
| 14 | Schaftabschnitt | | |
| 16 | erster Leiter | | |
| 18 | Arbeitselektrode | | |
| 20 | zweiter Leiter | | |
| 22 | Gegenelektrode | | |
| 24 | Leiter | | |
| 26 | Isolator | | |
| 28 | Verbindungsgeraden | | |
| 30 | Hüllrohr | | |
| 32 | Innenrohr | | |
| 34 | Halteelemente | | |
| 36 | Handgriff | | |
| 38 | Griffteil | | |
| 40 | Griffteil | | |
| 42 | Schlitten | | |
| 44 | Federbrücke | | |
| 46 | Gewebe | | |

## Patentansprüche

1. Bipolares Elektrodeninstrument (10) zur Verwendung in einem Resektoskop (12), wobei das Elektrodeninstrument (10) einen langgestreckten Schaftabschnitt (14) aufweist, durch den hindurch sich ein erster Leiter (16) erstreckt, der am distalen Ende des Elektrodeninstruments (10) eine mit Hochfrequenzstrom beaufschlagbare Arbeitselektrode (18) bildet, wobei sich ferner durch den Schaftabschnitt (14) ein zweiter Leiter (20) erstreckt, der im distalen Endbereich des Elektrodeninstruments (10) eine Gegenelektrode (22) bildet, wobei die Arbeitselektrode (18) und die Gegenelektrode (22) räumlich derart voneinander getrennt sind, dass der Abstand zwischen ihnen nicht mittels eines geraden Leiters (24) überbrückbar ist, der eine Länge von 6 mm oder weniger aufweist, und räumlich zwischen Arbeitselektrode (18) und Gegenelektrode (22) ein Isolator (26) angeordnet ist, der alle zwischen Arbeitselektrode (18) und Gegenelektrode (22) möglichen Verbindungsgeraden (28) schneidet, wobei der Isolator (26) einen L-förmigen Querschnitt aufweist und die Arbeitselektrode (18) zweiseitig von dem Isolator (26) umschlossen ist, **dadurch gekennzeichnet, dass** eine Seite, vorzugsweise zur Befestigung des Isolators (26),
die einem Tragarm (48) für die Arbeitselektrode (18) zugewandte Seite der Arbeitselektrode (18) ist und die andere Seite die Verbindungsgeraden (28) schneidet, wobei die Arbeitselektrode (18) am distalen Ende eines Tragarms (48) angeordnet ist, und wobei der Tragarm (48) in einem distalen Abschnitt, in dem die Arbeitselektrode (18) angeordnet ist, quer zur Längsachse des Elektrodeninstruments (10) verläuft und nach proximal abgewinkelt in einen Abschnitt des distalen Endbereichs übergeht, der parallel zur Längsachse des Elektrodeninstruments (10) angeordnet ist, und wobei in diesem Abschnitt, der parallel zur Längsachse ausgerichtet ist, die Gegenelektrode (22) angeordnet ist, die an der Außenhülle des Tragarms (48) angebracht ist.

2. Elektrodeninstrument (10) nach Anspruch 1, **dadurch gekennzeichnet, dass** der Abstand zwischen Arbeitselektrode (18) und Gegenelektrode (22) mehr als 6 mm beträgt, vorzugsweise mehr als 8 mm.

3. Elektrodeninstrument (10) nach einem der vorangehenden Ansprüche, **dadurch gekennzeichnet, dass** der Isolator (26) an die Arbeitselektrode (18) angrenzt.

4. Elektrodeninstrument (10) nach einem der vorangehenden Ansprüche, **dadurch gekennzeichnet, dass** der Isolator (26) plattenförmig ausgebildet ist.

5. Elektrodeninstrument (10) nach einem der vorangehenden Ansprüche, **dadurch gekennzeichnet, dass** die Arbeitselektrode (18) eine teilsphärische Vaporisationselektrode ist.

6. Resektoskop (12) für die endoskopische Chirurgie mit einem Hüllrohr (30), **dadurch gekennzeichnet, dass** innerhalb des Hüllrohres (30) ein Elektrodeninstrument (10) nach einem der Ansprüche 1 bis 5 längsverschiebbar gelagert ist.

## Claims

1. A bipolar electrode instrument (10) for use in a resectoscope (12), wherein the electrode instrument (10) has an elongated shaft section (14) through which a first conductor (16) extends and forms a working electrode (18) at the distal end of the electrode instrument (10), said working electrode (18) being configured to receive a high-frequency current, wherein furthermore a second conductor (20) extends through the shaft section (14) and forms a counter electrode (22) at the distal end region of the electrode instrument (10), wherein the working electrode (18) and the counter electrode (22) are spatially separated such that the distance between them cannot be bridged by a straight conductor (24) having a length of 6 mm or less, and an insulator (26) is arranged spatially between working electrode (18) and counter electrode (22), the insulator (26) intersecting all possible straight connecting lines (28) between working electrode (18) and counter electrode (22), wherein the insulator (26) has an L-shaped cross-section and the working electrode (18) is enclosed on two sides by the insulator (26), **characterized in that** one side, preferably for fastening the insulator (26), is the side of the working electrode (18) facing a support arm (48) for the working electrode (18) and the other side intersects the straight connecting lines (28), wherein the working electrode (18) is arranged at the distal end of a support arm (48), and wherein the support arm (48) extends transversely to the longitudinal axis of the electrode instrument (10) in a distal section in which the working electrode (18) is arranged and angled in proximal direction transitions into a section of the distal end region which is arranged parallel to the longitudinal axis of the electrode instrument (10), and wherein the counter electrode (22) is arranged in this section, which is aligned parallel to the longitudinal axis, and is attached to the outer shell of the support arm (48).

2. The electrode instrument (10) according to claim 1, **characterized in that** the distance between working electrode (18) and counter electrode (22) is greater than 6 mm, preferably greater than 8 mm.

3. The electrode instrument (10) according to one of the preceding claims, **characterized in that** the insulator (26) is adjacent to the working electrode (18).

4. The electrode instrument (10) according to one of the preceding claims, **characterized in that** the insulator (26) is plate-shaped.

5. The electrode instrument (10) according to one of the preceding claims, **characterized in that** the working electrode (18) is a partially spherical vaporization electrode.

6. A resectoscope (12) for endoscopic surgery with a cladding tube (30), **characterized in that** an electrode instrument (10) according to one of claims 1 to 5 is mounted so as to be longitudinally displaceable inside the cladding tube (30).

## Revendications

1. Instrument (10) à électrodes bipolaires destiné à être utilisé dans un résectoscope (12), l'instrument à électrodes (10) comprenant une partie de tige allongée (14) à travers laquelle s'étend un premier conducteur (16) qui forme, à l'extrémité distale de l'instrument à électrodes (10), une électrode de travail (18) apte à recevoir un courant à haute fréquence, un deuxième conducteur (20) s'étendant en outre à travers la partie formant tige (14) et formant une contre-électrode (22) dans la zone d'extrémité distale de l'instrument à électrodes (10), l'électrode de travail (18) et la contre-électrode (22) étant séparées spatialement l'une de l'autre de telle sorte que la distance entre elles ne puisse pas être franchie au moyen d'un conducteur droit (24) présentant une longueur de 6 mm ou moins, et un isolateur (26) étant agencé spatialement entre l'électrode de travail (18) et la contre-électrode (22), lequel isolateur coupe toutes les droites de connexion (28) possibles entre l'électrode de travail (18) et la contre-électrode (22), l'isolateur (26) présentant une section transversale en forme de L et l'électrode de travail (18) étant entourée sur deux côtés par l'isolateur (26), **caractérisé en ce qu'**un côté, de préférence pour la fixation de l'isolateur (26), est le côté de l'électrode de travail (18) tourné vers un bras de support (48) pour l'électrode de travail (18), et l'autre côté coupe les droites de connexion (28), l'électrode de travail (18) étant agencée à l'extrémité distale d'un bras de support (48), et le bras de support (48) s'étendant transversalement à l'axe longitudinal de l'instrument à électrodes (10) dans une partie distale dans laquelle est agencée l'électrode de travail (18) et se prolongeant de manière coudée vers le côté proximal dans une partie de la zone d'extrémité distale qui est agencée parallèlement à l'axe longitudinal de l'instrument à électrodes (10), et dans lequel, dans cette partie orientée parallèlement à l'axe longitudinal, est agencée la contre-électrode (22) qui est fixée à l'enveloppe extérieure du bras de support (48).

2. Instrument à électrodes (10) selon la revendication 1, **caractérisé en ce que** la distance entre l'électrode de travail (18) et la contre-électrode (22) est supérieure à 6 mm, de préférence supérieure à 8 mm.

3. Instrument à électrodes (10) selon l'une des revendications précédentes, **caractérisé en ce que** l'isolateur (26) est adjacent à l'électrode de travail (18).

4. Instrument à électrodes (10) selon l'une des revendications précédentes, **caractérisé en ce que** l'isolateur (26) est conçu sous la forme d'une plaque.

5. Instrument à électrodes (10) selon l'une des revendications précédentes, **caractérisé en ce que** l'électrode de travail (18) est une électrode de vaporisation partiellement sphérique.

6. Résectoscope (12) pour la chirurgie endoscopique avec un tube enveloppe (30), **caractérisé en ce qu'**un instrument à électrodes (10) selon l'une des revendications 1 à 5 est monté de manière longitudinalement déplaçable à l'intérieur du tube enveloppe (30).
